# EUROPEAN PATENT APPLICATION

(11) **EP 2 659 897 A1**
(43) Date of publication of application: **06.11.2013**
(21) Application number: 12166390.0
(22) Date of filing: 02.05.2012
(51) Int. Cl.: A61K 35/30, A61P 9/00

(54) **Stem cell derived composition for the treatment of acute injury and degenerative diseases**

(71) Applicant: PHILIPPS-UNIVERSITÄT MARBURG, 35037 Marburg (DE)
(72) Inventor: Culmsee, Carsten, 35075 Gladenbach (DE); Blomgren, Klas, Stockholm (SE); Pfeifer, Stefanie, 34132 Kassel (DE); Öxler, Eva-Maria, 35641 Schöffengrund (DE); Sato, Yoshiaki, Nagoya, 466-0063 (JP); Zhu, Changlian, 43638 Askim (SE)
(74) Representative: Stumpf, Peter

(57) **Abstract**

The invention comprises a composition obtained from dying pluripotent neural stem and progenitor cells undergoing cell death that is useful for the treatment of acute injury and degenerative diseases. Furthermore, a method for synthesis of the composition according to the invention is provided.

## Description

### Background of the invention

Intracerebral transplantation of neural stem and progenitor cells (NSPC) into injured or degenerating brain regions provides protective effects and functional regeneration in various models of acute brain damage or neurodegenerative diseases. Interestingly, such therapeutic approaches using NSPC also succeed, when the NSPC are applied with a significant delay of several days into the injured tissue. or even when applied systemically, e.g. into the blood stream. These promising results suggested that the applied NSPC had a pronounced capacity to replace injured and dead brain tissue. Most of the transplanted NSPC, however, die and disappear rapidly after placing into the brain tissue and only very few cells survive and transform into neural cells in the injured brain tissue. Similarly, only very few, if any of the NSPC injected systemically into the blood stream ever reach the injured brain tissue to transform into functional neurons or other neural cells. In addition,identified stem cell-derived new neurons after systemic application of mesenchymal stem cells more likely result from cell fusion than from exogenous neurogenesis. Thus, enhanced neurogenesis plays apparently a minor role for the observed protective effects after therapeutic mesenchymal NSPC applications as judged by morphological and functional analyses. Therefore, the initial concept of tissue replacement and significant generation of functional neural tissue by stem cell / NSPC therapy was extended by hypotheses suggesting that transplanted stem cells/NSPC mediate structural and functional neuroprotection by secretion of trophic factors that provide protective effects. Consequently, the NSPC were genetically modified in order to achieve sustained survival or/and enhanced expression and secretion of neurotrophic growth factors after transplantation. These attempts indeed prolonged the survival of the NSPC also enhanced the associated neuroprotective effects. However, the therapeutic effects were not as pronounced as expected, and neurogenesis and survival of the transplanted cells were only slightly enhanced. Further, transplantation of genetically modified stem cells and NSPC may significantly increase the risk of teratoma or tumor formation and adverse effects by the enhanced and uncontrolled secretion of neurotrophic growth factors. Therefore, the present invention follows an entirely novel concept that is not aiming to enhance survival and integration of the transplanted NSPC to enhance exogenous neurogenesis and tissue replacement. In contrast to these previous concepts, the current invention is based on death of the transplanted NSPC as a prerequisite for their therapeutic potential. Lethal stress and cell death of the NSPC leads to the secretion of a composition that provides neuroprotective effects in vitro and in vivo. Such neuroprotective effects are achieved, because the NSPC are dying and also application of a cell free composition obtained from dying stem cells / progenitor cells is sufficient to achieve neuroprotection.

### Technical problem

Only very few neural stem and progenitor cells (NSPC) survive transplantation into the brain which nevertheless provides measurable neuroprotective effects on structural and functional levels. Furthermore, no cellular or cell free compositions are available that are able to prevent acute or delayed dysfunction and death of neural cells after acute injury or during progression of neurodegenerative diseases. In fact, compositions providing neuroprotective effects are not available in the clinic to date. In order to overcome this important therapeutic gap a composition that is useful for treatment of acute neuronal injury, neurodegenerative diseases and cardiovascular diseases and a method for production of this composition are needed.

### Solution of the problem

The aforementioned technical problem is solved by a composition comprising proteins from pluripotent stem and progenitor cells that are dying by cell death. Surprisingly, it is found in the invention that proteins from these dying cells mediate a neuroprotective effect wherein death of the cells is a prerequisite to provide neuroprotection more effectively than a crude cell extract of stem or progenitor cells growing under standard culture conditions. Notably, heating of the composition further increases the neuroprotective effect. Furthermore, a method is provided for the synthesis of the composition.

### Description of the invention

The present invention relates to a composition comprising proteins from pluripotent stem cells or neural progenitor cells undergoing cell death.

Preferably, the composition is derived from cell lysate or a conditioned medium of pluripotent stem cells or neural progenitor cells dying by cell death.

Lysis refers to the breaking down of a cell, for example by viral, enzymatic, or osmotic mechanisms that compromise the cell's integrity. A fluid containing the contents of lysed cells is called cell lysate.

Conditioned medium is medium harvested from cultured cells under standard culturing conditions or at defined time points after induction of cell death. It contains metabolites and proteins such as growth factors, and extracellular matrix proteins that are secreted into the medium by the cultured cells. Examples of each might include: metabolites such as glucose, amino acids, and nucleosides; growth factors such as interleukins, EGF (epidermal growth factor), and PDGF (platelet-derived growth factor); and matrix proteins such as collagen, fibronectin, and various proteoglycans.

Most preferably, the proteins of the composition are selected from the group of peroxiredoxins, galectins, heat shock proteins, transcription factors, and/or translation factors.

Peroxiredoxins (Prdx, EC 1.11.1.15) are a ubiquitous family of antioxidant enzymes that also control cytokine-induced peroxide levels and thereby mediate signal transduction in mammalian cells. Peroxiredoxins can be regulated by changes to phosphorylation, redox and possibly oligomerization states. They are divided into three classes: typical 2-Cys Prdx, atypical 2-Cys Prdx, and 1-Cys Prdx. These enzymes share the same basic catalytic mechanism, in which a redox-active cysteine (the peroxidatic cysteine) in the active site is oxidized to a sulfenic acid by the peroxide substrate. The recycling of the sulfenic acid back to a thiol is what distinguishes the three enzyme classes: 2-Cys peroxiredoxins are reduced by thiols such as glutathione, while the 1-Cys enzymes may be reduced by ascorbic acid or glutathione in the presence of GST-π (GST Pi). Using crystal structures, a detailed catalytic cycle has been derived for typical 2-Cys Prdx, including a model for the redox-regulated oligomeric state proposed to control enzyme activity. Inactivation of these enzymes by over-oxidation of the active thiol to sulfinic acid can be reversed by sulfiredoxin. The physiological importance of peroxiredoxins is illustrated by their relative abundance (one of the most abundant proteins in erythrocytes after hemoglobin is peroxiredoxin 2) as well as studies in knockout mice. Mice lacking peroxiredoxin 1 or 2 develop severe haemolytic anemia, and are predisposed to certain haematopoietic cancers. Peroxiredoxin 1 knockout mice have a 15% reduction in lifespan. Peroxiredoxin 6 knockout mice are viable and do not display obvious gross pathology, but are more sensitive to certain exogenous sources of oxidative stress, such as hyperoxia. Peroxiredoxin 3 (mitochondrial matrix peroxiredoxin) knockout mice are viable and do not display obvious gross pathology. Peroxiredoxins are frequently referred to as alkyl hydroperoxide reductase (AhpC) in bacteria. Other names include thiol specific antioxidant (TSA). This family contains AhpC and TSA, as well as related proteins. Mammals express six peroxiredoxins: 1-Cys enzymes: PRDX6; 2-Cys enzymes: PRDX1, PRDX2, PRDX3, PRDX4, and PRDX5.

In a particular preferred embodiment, the peroxiredoxin is peroxiredoxin-1. Peroxiredoxin-1 is a protein that in humans is encoded by the PRDX1 gene. This gene encodes a member of the peroxiredoxin family of antioxidant enzymes, which reduce hydrogen peroxide and alkyl hydroperoxides. The encoded protein may play an antioxidant protective role in cells, and may contribute to the antiviral activity of CD8(+) T-cells. Furthermore, this protein may have a proliferative effect and play a role in cancer development or progression. Three transcript variants encoding the same protein have been identified for this gene.

Galectins, also known as galaptins or S-lectin, are a family of lectins which bind beta-galactoside. The family is defined by having at least one characteristic carbohydrate recognition domain (CRD) with an affinity for beta-galactosides and sharing certain sequence elements. Members of the galectin family are found in mammals, birds, amphibians, fish, nematodes, sponges, and some fungi. Galectins are known to carry out intra- and extracellular functions, including inhibition of chronic inflammations, GVHD, and allergic reactions, through glycoconjugate-mediated recognition. From the cytosol they may be secreted by non-classical pathways, but they may also be targeted to the nucleus or specific sub-cytosolic sites. Within the same peptide chain some galectins have a CRD with only a few additional amino acids, whereas others have two CRDs joined by a link peptide, and one (galectin-3) has one CRD joined to a different type of domain. Genes encoding galectins include: LGALS1, LGALS2, LGALS3, LGALS4, LGALS5, LGALS6, LGALS7, LGALS8, LGALS9, LGALS12, LGALS13.

In a particular preferred embodiment, the galectin is galectin-1. Galectin-1 is a protein that in humans is encoded by the LGALS1 gene. Galectin-1 is thought to play a role in the immunosuppression required for a successful pregnancy. Galectin-1 induces the differentiation of dendritic cells towards a phenotype which dampens T helper 1 cells and T helper 17 cells and dampens inflammation via interleukin-10 and interleukin-27.

Heat shock proteins (HSP) are a class of functionally related proteins involved in the folding and unfolding of other proteins. Their expression is increased when cells are exposed to elevated temperatures or other stress. This increase in expression is transcriptionally regulated. The dramatic upregulation of the heat shock proteins is a key part of the heat shock response and is induced primarily by heat shock factor (HSF). HSPs are found in virtually all living organisms, from bacteria to humans. Production of high levels of heat shock proteins can also be triggered by exposure to different kinds of environmental stress conditions, such as infection, inflammation, exercise, exposure of the cell to toxins (ethanol, arsenic, trace metals, and ultraviolet light, among many others), starvation, hypoxia (oxygen deprivation), nitrogen deficiency (in plants), or water deprivation. As a consequence, the heat shock proteins are also referred to as stress proteins and their upregulation is sometimes described more generally as part of the stress response. Heat shock proteins function as intra-cellular chaperones for other proteins. They play an important role in protein-protein interactions such as folding and assisting in the establishment of proper protein conformation (shape) and prevention of unwanted protein aggregation. By helping to stabilize partially unfolded proteins, HSPs aid in transporting proteins across membranes within the cell. Heat-shock proteins also occur under non-stressful conditions, simply monitoring the cell's proteins. Some examples of their role as monitors are that they carry old proteins to the cell's "recycling bin" (proteasome) and that they help newly synthesized proteins fold properly. These activities are part of a cell's own repair system, called the "cellular stress response" or the "heat-shock response". Heat shock proteins appear to serve a significant cardiovascular role. Hsp90, hsp84, hsp70, hsp27, hsp20, and alpha B crystallin all have been reported as having roles in the cardiovasculature. Hsp90 binds both endothelial nitric oxide synthase and soluble guanylate cyclase, which in turn are involved in vascular relaxation. A downstream kinase of the nitric oxide cell signalling pathway, protein kinase G, phosphorylates a small heat shock protein, Hsp20. Hsp20 phosphorylation correlates well with smooth muscle relaxation and is one significant phosphoprotein involved in the process. Hsp20 appears significant in development of the smooth muscle phenotype during development. Importantly, Hsp20 also serves a significant role in preventing platelet aggregation, cardiac myocyte function and prevention of apoptosis after ischemic injury, and skeletal muscle function and muscle insulin response. Extracellular and membrane bound heat-shock proteins, especially Hsp70 are involved in binding antigens and presenting them to the immune system.

Transcription factors, also called sequence-specific DNA-binding factors, are proteins that bind to specific DNA sequences, thereby controlling the flow or transcription of genetic information from DNA to mRNA. Transcription factors perform this function alone or with other proteins in a complex, by promoting as an activator, or blocking as a repressor the recruitment of RNA polymerase, i.e. the enzyme that performs the transcription of genetic information from DNA to RNA, to specific genes. A defining feature of transcription factors is that they contain one or more DNA-binding domains (DBDs), which attach to specific sequences of DNA adjacent to the genes that they regulate.

Translation factors are proteins that are needed for the translation process by which messenger RNA is translated into proteins in eukaryotes. Translation consists of initiation, elongation and termination and is dependent on the translation factors comprising initiation factors, elongation factors and release factors.

Eukaryotic initiation factors (eIF) are proteins involved in the initiation phase of eukaryotic translation. They function in forming a complex with the 40S ribosomal subunit and Met-tRNAᵢ called the 43S preinitation complex (PIC), recognizing the 5' cap structure of mRNA and recruiting the 43S PIC to mRNA, promoting ribosomal scanning of mRNA and regulating recognition of the AUG initiation codon, and joining of the 60S ribosomal subunit to create the 80S ribosome. The protein RLI is known to have an essential, probably catalytic role in the formation of initiation complexes as well.

Elongation in eukaryotes is carried out with two elongation factors: eEF-1 and eEF-2. eEF-1 has two subunits, α and βγ. α mediates the entry of the aminoacyl tRNA into a free site of the ribosome. βγ serves as the guanine nucleotide exchange factor for α, catalyzing the release of GDP from α. eEF-2 catalyzes the translocation of the tRNA and mRNA down the ribosome at the end of each round of polypeptide elongation. Eukaryotic translation termination factor 1 (eRF1), also known asTB3-1, is a protein that in humans is encoded by the *ETF1* gene.

In eukaryotes, the release factor eRF recognizes all three stop codons. Eukaryotic translation termination factor 1 (eRF1), also known as TB3-1, is a protein that in humans is encoded by the *ETF1* gene. Termination of protein biosynthesis and release of the nascent polypeptide chain are signaled by the presence of an in-frame stop codon at the aminoacyl site of the ribosome. The process of translation termination is universal and is mediated by protein release factors (RFs) and GTP.

### Manufacturing of the composition according to the invention

According to the present invention, the composition as referred to herein is produced by a method comprising the steps:
i. cultivation of pluripotent neural stem and progenitor cells in appropriate cell culture medium,
ii. induction of cell death,
iii. harvest and lysis of cells or harvest of conditioned medium.

Cultivation of pluripotent neural stem and progenitor cells according to the present invention is performed to state of the art techniques using common cultivation media, like e.g. Dulbecco's modified Eagle's medium (DMEM) or neurobasal medium that are supplemented with essential growth factors, respectively, and are appropriate for this purpose. These growth factors comprise for example epidermal and basic fibroblast growth factors.

Pluripotent stem cells have the potential to differentiate into any of the three germ layers: endoderm (interior stomach lining, gastrointestinal tract, the lungs), mesoderm (muscle, bone, blood, urogenital), or ectoderm (epidermal tissues and nervous system). Pluripotent stem cells can give rise to any fetal or adult cell type. Neural progenitor cells are cells that, like stem cells, have a tendency to differentiate into neural cells. These cells are more specific than stem cells and are pushed to differentiate into their target cell. The most important difference between stem cells and progenitor cells is that stem cells can replicate indefinitely, whereas progenitor cells can divide only a limited number of times. The terms "progenitor cell" and "stem cell" are sometimes equated.

According to the present invention, induction of cell death is induced after cultivation of cells for at least 24 hours. Preferably, induction is performed by state of the art methods like for example growth factor deprivation, wherein the cultivation medium is replaced with a medium lacking epidermal and basic fibroblast growth factors. Alternatively, induction of cell death is induced by hypoxia, oxygen-glucose deprivation, death receptor ligands, pro-apoptotic compounds and toxins, irradiation, DNA-damage, oxidative stress, mechanical injury, or heat shock, respectively.

The composition as referred to herein comprising proteins of pluripotent stem cells or neural progenitor cells, respectively, that die by cell death, is then gained either by harvest of the spent cultivation medium, called conditioned medium, or harvest and lysis of the dying cells, respectively, by state of the art methods. Cells are lysed for example by mechanical lysis, for example by a cell douncer, or by sonication. The composition may also be harvested at lower potency after lysis of stem cells or progenitor cells cultured under standard cell culture conditions, i.e. without induction of cell death.

Alternatively, another step iv. comprising filtration of cell lysate or conditioned medium with a common 0.22 µm membrane filter follows step iii. according to the invention as described herein in order to sterilize the composition.

Alternatively, another step v. comprising filtration with a filter having a size exclusion of at least 3 kDa follows step iii. or iv., respectively, according to the invention as described herein in order to concentrate effective proteins of the composition.

Also alternatively, another step vi. comprising heating of the composition to between 20°C or up to 95°C for at least 10 minutes follows step iii, iv. and/or step v., respectively, in order to enhance effectivity of the composition. Preferably, the composition is heated to between 50°C or up to 70°C. Most preferably, the filtrate is heated to 60°C.

Also alternatively, another step vii. comprising precipitation, fractionation, dialysis and/or lyophilisation of the composition follows step iii., iv., v., and/or vi., respectively.

### Use of the composition according to the invention

The composition according to the present invention is used for the treatment of acute injury or degenerative diseases of humans or animals. The composition is administered to patients as an oral, rectal, parenteral, intravenous, intramuscular, subcutaneous, intracisternal, intravaginal, intraperitoneal, intravascular, local, or inhalative formulation.

Acute injury as referred to herein is caused by mechanical impact, ischemic injury or by irradiation.

The degenerative disease as referred to herein is a neurodegenerative disease of the peripheral nerve system or the central nerve system, a cardiovascular disease, diabetes-related degenerative processes or diseases affecting the kidneys.

A degenerative disease is a disease in which the function or structure of the affected tissues or organs will progressively deteriorate over time. Examples of degenerative diseases are: Amyotrophic Lateral Sclerosis (ALS), Alzheimer's disease, Parkinson's Disease, Multiple system atrophy, Niemann Pick disease, Atherosclerosis, Progressive supranuclear palsy, Tay-Sachs Disease, Diabetes, Heart Disease, Keratoconus, Inflammatory Bowel Disease (IBD), Prostatitis, Osteoarthritis, Osteoporosis, Rheumatoid Arthritis, Huntington's Disease, Chronic traumatic encephalopathy, Epilepsy, Dementia, Renal failure, Multiple sclerosis, Malaria with CNS degeneration, Neuro-AIDS, Lysosomal storage diseases, Encephalatis of viral, bacterial or autoimmune origin.

### Examples

The examples below illustrate the present invention. However, they shall not be regarded as scope limiting.

### Example 1: Cultivation of pluripotent neural stem and progenitor cells (NSPC) and control cells

According to the invention herein, pluripotent neuronal stem and progenitor cells (NSPC) (Ray J & Gage FH (2006) Mol Cell Neurosci 31, 560-573.) are cultured in state of the art Dulbecco's modified medium Ham's F12 (DMEM/F12; PAA, Cölbe, Germany) supplemented with 1% N2, 100U/ml penicillin, 100µg/ml streptomycin, 2mM glutamine, 10µg/ml epidermal growth factor, 10µg/ml basic fibroblast growth factor (all Invitrogen, Karlsruhe, Germany), and 5 mg/ml heparin (Sigma-Aldrich, Taufkirchen, Germany). NSPC are plated at a density of 60,000 cells/ cm² on E-plates 96 (Roche, Penzberg, Germany) or on regular 24 or 96 well plates (Greiner, Frickenhausen, Germany).

Immortalized HT-22 mouse hippocampal neurons (Li Y, Maher P, Schubert D. A role for 12-lipoxygenase in nerve cell death caused by glutathione depletion. Neuron. 1997;19:453-463) are maintained in DMEM (PAA, Cölbe, Germany). DMEM is supplemented with FBS (10%; Invitrogen, Karlsruhe, Germany), penicillin/streptomycin (100 x 1%; Invitrogen, Karlsruhe, Germany) and glutamine (100 x 1%; PAA, Cölbe, Germany). HT-22 cells are plated at a density of 20,000 cells/ cm² on regular 96-well plates (Greiner, Frickenhausen, Germany).

Primary cortical neurons (PCN) are prepared from cortices of embryonic rats (E 16-18) (Sprague Dawley, Janvier), as previously described (Culmsee C, Gerling N, Lehmann M, Nikolova-Karakashian M, Prehn JH, Mattson MP, Krieglstein J. Nerve growth factor survival signaling in cultured hippocampal neurons is mediated through TrkA and requires the common neurotrophin receptor P75. Neuroscience. 2002;115:1089-1108.; Culmsee C, Zhu C, Landshamer S, Becattini B, Wagner E, Pellecchia M, Blomgren, K, Plesnila N. Apoptosis-inducing factor triggered by poly(ADP-ribose) polymerase and Bid mediates neuronal cell death after oxygen-glucose deprivation and focal cerebral ischemia. J Neurosci. 2005; 25:10262-10272.). Briefly, meninges are removed and neurons are separated by mechanical dissociation after mild trypsinisation of the isolated cortical tissue. Cortical cells are plated at a density of 7x10⁵ cells/cm² on polyethylenimine-pre-coated 96-well plates or 35 mm culture dishes (both from Greiner, Frickenhausen, Germany). Primary neurons are cultured in state of the art neurobasal medium (Invitrogen, Karlsruhe, Germany), supplemented with Hepes (5 mM; Sigma-Aldrich, Taufkirchen, Germany), glutamine (1.2 mM; PAA, Cölbe, Germany), B27 supplement (2%, v/v; Invitrogen, Karlsruhe, Germany) and gentamicin (0.1 mg/ml; Sigma-Aldrich, Taufkirchen, Germany). After 48 hours, neurons are treated with cytosine arabinofuranosid for another 48 hours to block growth of astrocytes in the neuronal cultures. All experimental treatments are performed on 6 to 8 day-old cultures.

As control cells, mouse embryonic fibroblasts (MEF) are prepared from C57BL/6 mice on gestation day 18. The MEF are transferred to culture flasks containing Dulbecco's modified Eagle's medium (DMEM) high glucose (4.5 g/L) supplemented with FBS (fetal bovine serum; 15%), glutamine (100 x 2%) (all PAA, Cölbe, Germany) penicillin/streptomycin (100 x 1%; Invitrogen, Karlsruhe, Germany), and sodium-pyruvate (1 mM; Sigma- Aldrich, Taufkirchen, Germany). After attaching to the plastic the MEF divide within 1 to 3 days. Passages 1 to 3 are used for further experiments and plated at a density of 20,000 cells/ cm² (Xu J. Preparation, culture, and immortalization of mouse embryonic fibroblasts. Curr Protoc Mol Biol. 2005 Chapter 28:Unit 28.1).

All cell types are incubated at 37°C in a humidified incubator containing 5% CO₂.

### Example 2: Production of composition comprising cell lysate or conditioned medium from cells of example 1 dying by cell death and precipitated proteins thereof

Neural stem and progenitor cells (NSPC) or HT-22 cells, respectively, are used for the production of cell lysate or conditioned medium; SNL feeder cells or MEF, respectively, are used as control cells. For growth factor deprivation, medium is removed and cells are washed once with phosphate-buffered saline (PBS) when a cell confluence of about 70% is reached, and cells are incubated with state of the art Earle's balanced salt solution (EBSS; Sigma-Aldrich, Taufkirchen, Germany). This conditioned medium was used for later applications in HT-22, MEF and SNL cells. For later application in PCN conditioned medium produced in DMEM/Ham's F12 (PAA, Cölbe, Germany) supplemented with N2 (1 %; Invitrogen, Karlsruhe, Germany) deprived of epidermal growth factor (EGF) and basic fibroblast growth factor (bFGF) is used.

Cells are harvested by standard methods and lysed by ultrasound or conditioned medium (CM) is collected after 24 hours of growth factor deprivation from NSPC, or after 48 hours from HT-22 cells, or after 6 to 8 days from MEF and SNL cells, respectively. The cell lysate or conditioned medium is stored at -80°C until further use. To remove dead cells and cell debris, cell lysate or CM is centrifuged at 1,000 rpm for 10 minutes and filtered through a 0.22 µm membrane filter.

Alternatively, for acetone precipitation of proteins, cell lysate or fresh CM is mixed with the 4-fold amount of cold acetone (Roth, Karlsruhe, Germany) and kept at -20°C over night. The next day the solution is centrifuged at 13,000 x g and 4°C for 30 minutes. Supernatant is removed and the pellet containing the proteins is resuspended in EBSS.

Alternatively, for heat activation, cell lysate, CM, or precipitated proteins in EBSS, respectively, is incubated at 20°C or up to 95°C for at least 10 minutes directly before use.

### Evaluation of cell viability and apoptosis

Cell viability is evaluated 18 to 24 hours after growth factor withdrawal. Quantification of cell viability in NSPC and HT-22 cells is performed in 96-well plates by state of the art MTT (3-(4,5-dimethylthiazol-2-yl)-2,5-diphenyltetrazolium bromide) (Sigma-Aldrich, Taufkirchen, Germany) reduction at 0.25 mg/ml for 1 h. The reaction is terminated by removing the media from the cells and freezing the plate at -80°C for at least 1 h. Absorbance is determined after solving the MTT dye in dimethyl sulfoxide (DMSO; Sigma-Aldrich, Taufkirchen, Germany) at 570 versus 630 nm (FluoStar, BMG Labtech, Offenbach, Germany).

In addition, real-time detection of cellular viability is performed by cellular impedance measurements using the xCELLigence system (Roche Diagnostics, Penzberg, Germany) as described in the state of the art (Diemert et al., 2012). In brief, impedance measurements are carried out in 96-well E-plates (Roche Diagnostics, Penzberg, Germany). Background impedance caused by the media is determined in each well before seeding the cells and subtracted automatically by the RTCA software.

For quantification of pyknotic nuclei in primary cortical neurons exposed to growth factor withdrawal, cells are incubated according to common methods with 4',6-Diamidine-2-phenylindole (DAPI; 1µg/ml). Stained nuclei are visualized under epifluorescence illumination using a 20x objective. Neurons with condensed and fragmented nuclei are considered dead. About 1,000 cells per treatment condition are counted, and counts are made in five separate cultures without knowledge of the treatment history.

Fluorescence images are obtained using a DM16000 B inverted microscope (Leica, Germany) and analyzed using LAS AF Software (Leica Application Suite, Advanced Fluorescence 2.2.0, Leica Microsystems, Mannheim, Germany). For morphological analysis of NSPC, transmission light microscopy of NSPC growing as monolayers is performed using an Axiovert 200 microscope (Zeiss, Jena, Germany) equipped with a Sony DSC-S75 digital camera (Sony Corporation, Tokyo, Japan).

The experiments are each performed at least three times. All data points are given as means ± standard deviation (SD). Statistical comparisons between groups are performed with ANOVA, followed by Scheffé's or Duncan's test as indicated. A p value of < 0.05 is considered to indicate significant differences of the compared mean values. Calculations are performed with the Winstat standard statistical software package.

### Example 3: Treatment of growth factor deprived and glutamate-induced cells with composition from example 2

Immortalized HT-22 mouse hippocampal neurons, neural stem and progenitor cells (NSPC), or primary cortical neurons (PCN), respectively, are pre-treated with cell lysate, conditioned medium (CM), or precipitated proteins, respectively, corresponding to a composition from example 2 for at least 6 hours. Alternatively, heat activated composition according to example 2 is used for pretreatment of the cells. Afterwards, glutamate (2 or up to5 mM) is added to the cells for 18 hours. Glutamate addition serves as a model system for neurodegeneration mediated by oxidative stress and calcium deregulation. In particular, in HT-22 cells millimolar concentrations of glutamate (usually 2 - 5 mM) induce NMDA-receptor independent cell death through enhanced oxidative stress, mitochondrial damage and the release of mitochondrial apoptosis-inducing factor, a process termed oxytosis, . Alternatively, in primary cortical neurons (PCN), excitotoxicity and calcium-triggered cell death through activation of NMDA receptors is induced by micromolar concentrations of glutamate (10 or up to 50 µM).

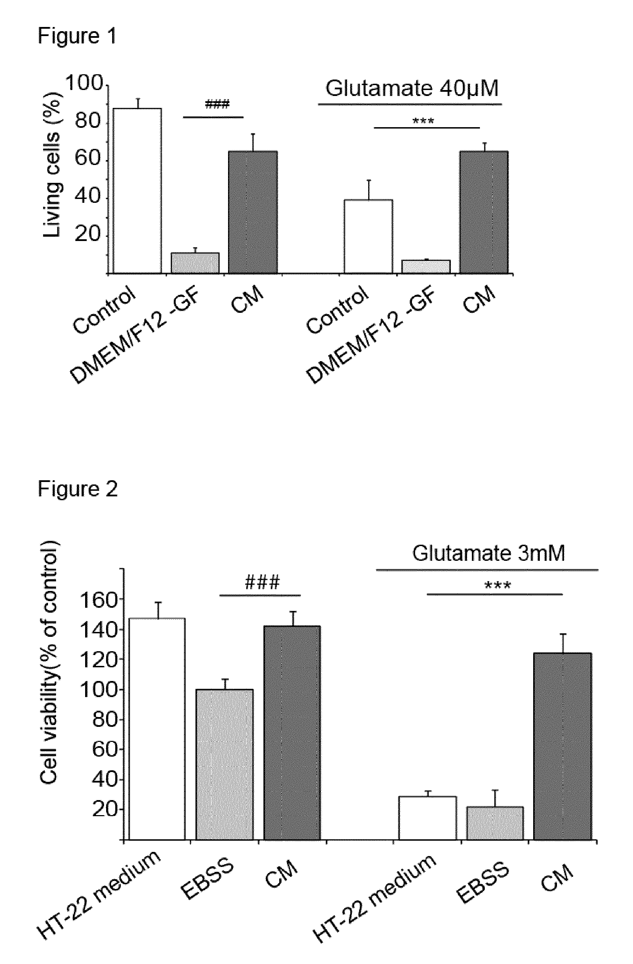

Growth factor deprivation and glutamate treatment of HT-22 cells or cultured PCN, respectively, leads in both cases to cellular death. Treatment with conditioned medium harvested from dying NSPC (NSPC-CM) or dying HT-22 cells (HT-CM), respectively, as referred to in example 2 elicits a neuroprotective effect against growth factor deprivation and glutamate-toxicity in both HT-22 and/or PCN cells (Figures 1, 2, and 3), indicating that dying NSPC produce protective factors that attenuate growth factor withdrawal and glutamate-induced neurotoxicity.

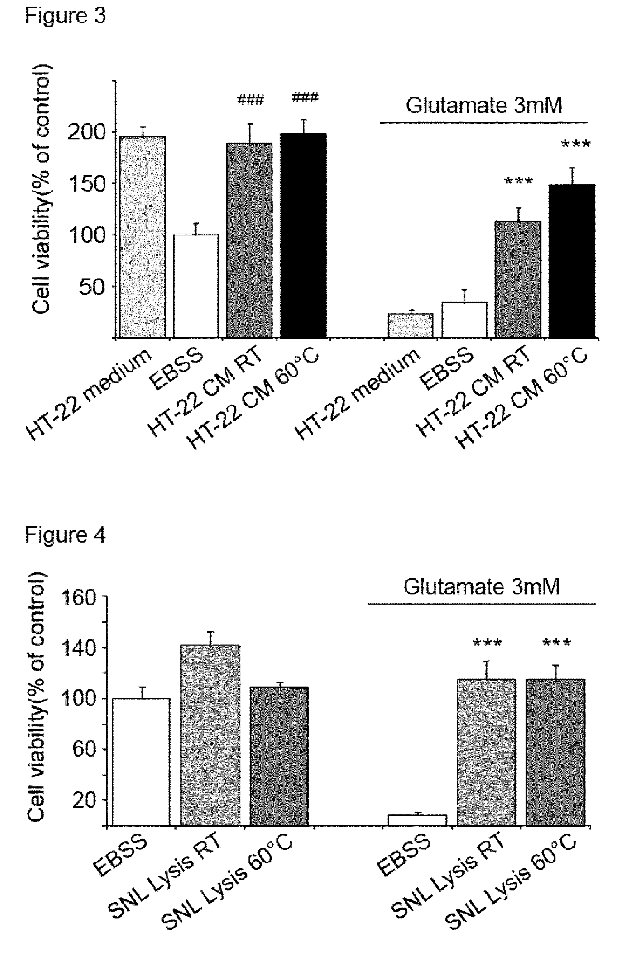

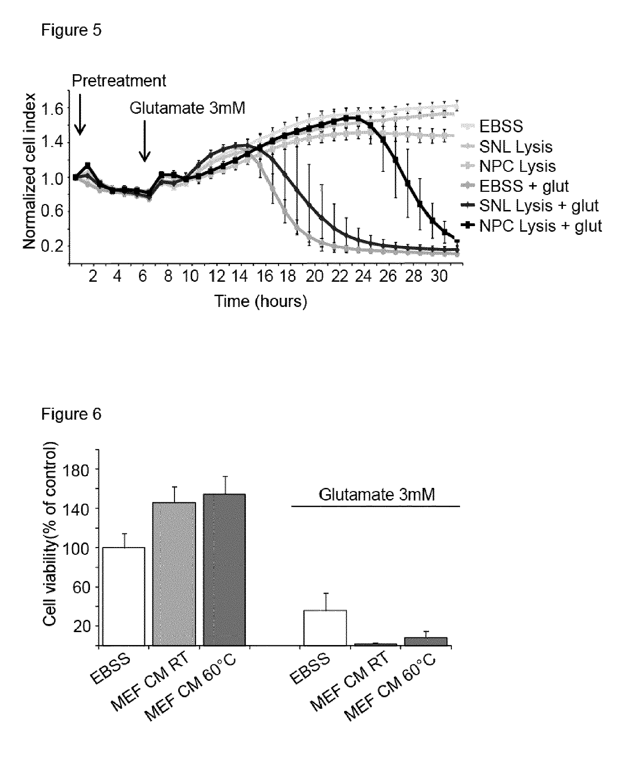

As shown by MTT assays, also cell lysates are able to protect against glutamate-induced cell death, suggesting that cell death of NSPC is necessary to release protective factors into the medium. Furthermore, a composition corresponding to SNL cell lysates obtained by sonication provides neuroprotection but only for very short time (Figure 4, 5).

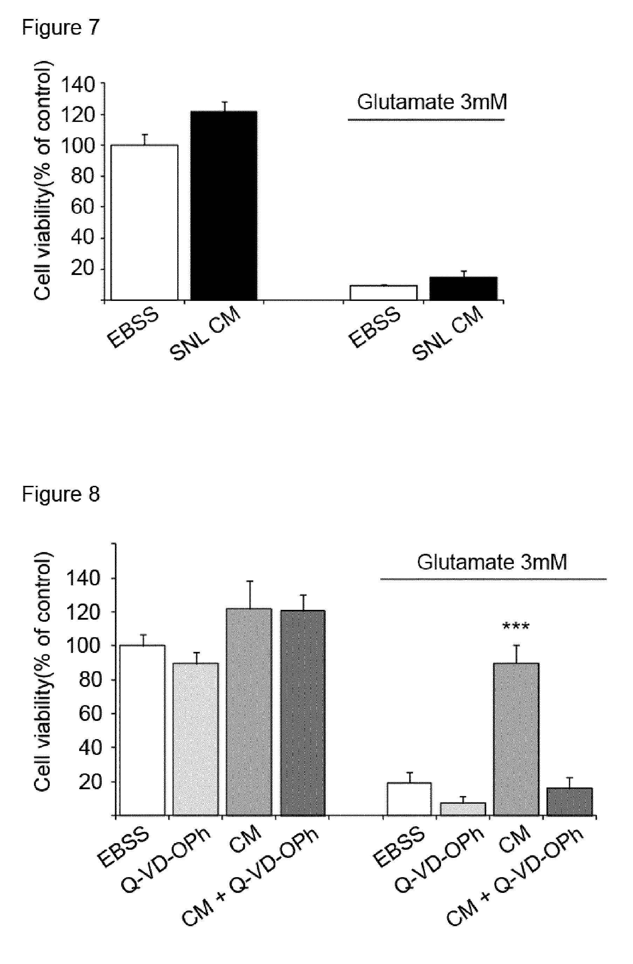

As a control, conditioned medium corresponding to a composition according to example 2 obtained from MEF or SNL cells after growth factor withdrawal is used. The respective conditioned media are applied to glutamate-exposed HT-22 cells similar to the experiments with NSPC-CM. Notably, CM from apoptotic MEF and SNL cells show no significant protective effect against glutamate-induced neurotoxicity in HT-22 cells (Figure 6, 7).

As a further control to test whether living cells are also able to release protective substances, for inhibition of cell death the broad caspase inhibitor Q-VD-OPh (QVD; Calbiochem, Darmstadt, Germany) is administered to the medium of cultured NSPC at a final concentration of 20 up to 40 µM. Dimethyl sulfoxide (DMSO; Sigma-Aldrich, Taufkirchen, Germany) is used as a standard solvent and vehicle for QVD. As known from the state of the art, QVD attenuates NSPC death after growth factor withdrawal. Notably, CM obtained from NSPC preserved by QVD fail to prevent cell death induced by glutamate in HT-22 cells (Figure 8). Importantly, QVD alone does not protect HT-22 cells from glutamate, as known from the state of the art.

As a further control, for protein digestion of the composition protease (Qiagen, Hilden, Germany) is used in a concentration of 45 mAU (AU: Anson Units) per mg protein. Digestion is done at 37°C for 30 minutes. Afterwards protease is inactivated by heating up to 70°C for at least 1 hour. Then the composition is used for treatment of growth factor deprived and glutamate-induced cells.

Treatment of the composition with protease shows a very strong reduction in cell viability suggesting that the protective protein factors are degraded and protein fragments rather accelerate the glutamate toxicity (Figure 9). Since proteases may exert unspecific effects, acetone precipitation with conditioned medium (CM) is performed to verify that the protective effect results from proteins. The precipitated proteins obtained after centrifugation of acetone-incubated CM are still active and provide significant neuroprotection (Figure 10). Furthermore, the protective effect of this acetone-precipitated protein solution is further activated by heat (Figure 11).

As a control for exclusion of RNA effects, RNA digestion of the composition is performed with 70 K units (K units: Kunitz Units) Ribonuclease A (Sigma-Aldrich, Taufkirchen, Germany) per mg protein. To get an optimal digestion, cell lysate, CM, or precipitated proteins and ribonuclease are kept at 60°C for 30 minutes resulting in inactivation of the RNAse Afterwards the composition is used for treatment of growth factor deprived and glutamate-induced cells.

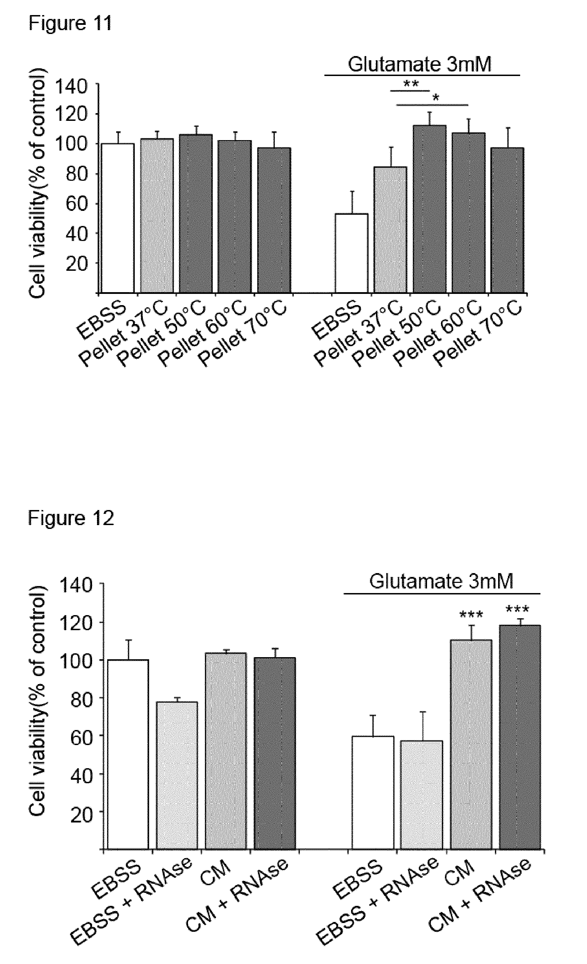

RNAse treatment of the composition shows no change in the neuroprotective effect of conditioned medium (CM), which excludes RNA as protective component of CM (Figure 12).

### Treatment with heated composition

Alternatively, according to example 2 the composition was heated to 20°C or up to 95°C for at least 10 minutes before treating the growth factor deprived and glutamate-induced cells with the composition. For the treatment the heated composition is used undiluted or as diluted up to 1:4 with EBSS. Dilutions of the compositions higher than 1:4 do not provide significant protective effects.

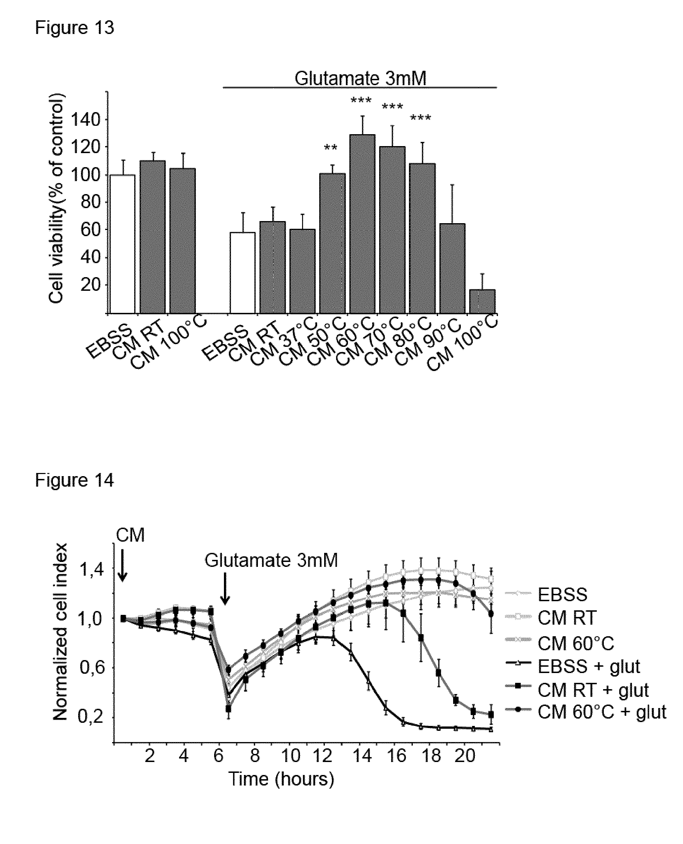

The most significant and strong neuroprotective effect is found with composition that is heated to 60°C (Figure 13). Real time analyses performed with by impedance measurements confirm that NSPC-CM heated to 60°C is more potent than native NSPC-CM (Figure 14). Higher temperatures of more than 90°C destroy the protective effect (Figure 13).

### Evaluation of cell viability and apoptosis

Cell viability is evaluated 12 to 18 hours after glutamate treatment. Quantification of cell viability in NSPC and HT-22 cells is performed in 96-well plates by MTT (3-(4,5-dimethylthiazol-2-yl)-2,5-diphenyltetrazolium bromide) (Sigma-Aldrich, Taufkirchen, Germany) reduction at 0.25 mg/ml for 1 h. The reaction is terminated by removing the media from the cells and freezing the plate at -80°C for at least 1 h. Absorbance is determined after solving the MTT dye in dimethyl sulfoxide (DMSO; Sigma-Aldrich, Taufkirchen, Germany) at 570 versus 630 nm (FluoStar, BMG Labtech, Offenbach, Germany).

In addition, real-time detection of cellular viability is performed by cellular impedance measurements using the xCELLigence system (Roche Diagnostics, Penzberg, Germany) as described previously (Diemert S, Dolga AM, Tobaben S, Grohm J, Pfeifer S, Oexler E, Culmsee C. Impedance measurement for real time detection of neuronal cell death. J Neurosci Methods. 2012; 203:69-77). In brief, impedance measurements are carried out in 96 well E-plates (Roche Diagnostics, Penzberg, Germany). Background impedance caused by the media is determined in each well before seeding the cells and subtracted automatically by the RTCA software.

For quantification of pyknotic nuclei in primary cortical neurons exposed to glutamate, cells are incubated with DAPI (1µg/ml). Stained nuclei are visualized under epifluorescence illumination using a 20x objective. Neurons with condensed and fragmented nuclei are considered apoptotic. About 1000 cells per treatment condition are counted, and counts are made in five separate cultures without knowledge of the treatment history.

Fluorescence images are obtained using a DMI 6000 B inverted microscope (Leica, Germany) and analyzed using LAS AF Software (Leica Application Suite, Advanced Fluorescence 2.2.0, Leica Microsystems, Mannheim, Germany). For morphological analysis of NSPC, transmission light microscopy of NSPC growing as monolayers is performed using an Axiovert 200 microscope (Zeiss, Jena, Germany) equipped with a Sony DSC-S75 digital camera (Sony Corporation, Tokyo, Japan).

The experiments are each performed at least three times. All data points are given as means ± standard deviation (SD). Statistical comparisons between groups are performed with ANOVA, followed by Scheffé's or Duncan's test as indicated. A p value of < 0.05 is considered to indicate significant differences of the compared mean values. Calculations are performed with the Winstat standard statistical software package.

### Example 4: Identification of neuroprotective proteins

Conditioned medium from example 2 is used as sample. Samples are concentrated by centrifugation through a 10 kDa cut-off filter (Amicon, Schorndorf, Germany) at 4,300 U/min. Samples are digested with 1 M NH₄HCO₃, acetonitrile, 500 mM DTT and 7,4 µg/ml trypsine in 1 mM HCl/15mM CaCl₂ (Promega). After digestion the samples are acidified by acetic acid and are investigated by MALDI-TOF analyses performed on an ABI 4800 Proteomics Analyzer (Applied Biosystems, Framingham, MA). The fragmentation patterns are used to manually determine the sequence of the peptides by using the PubMed database.

The list of components in NSPC-CM comprises a spectrum of different proteins including stress response proteins, antioxidative factors and molecular chaperones. Among these, peroxiredoxins, heat shock proteins and galectins are the most promising candidates mediating protective effects against cell degeneration and death. In addition, proteins mediating DNA repair are identified in the CM (see sequence listing for list of components).

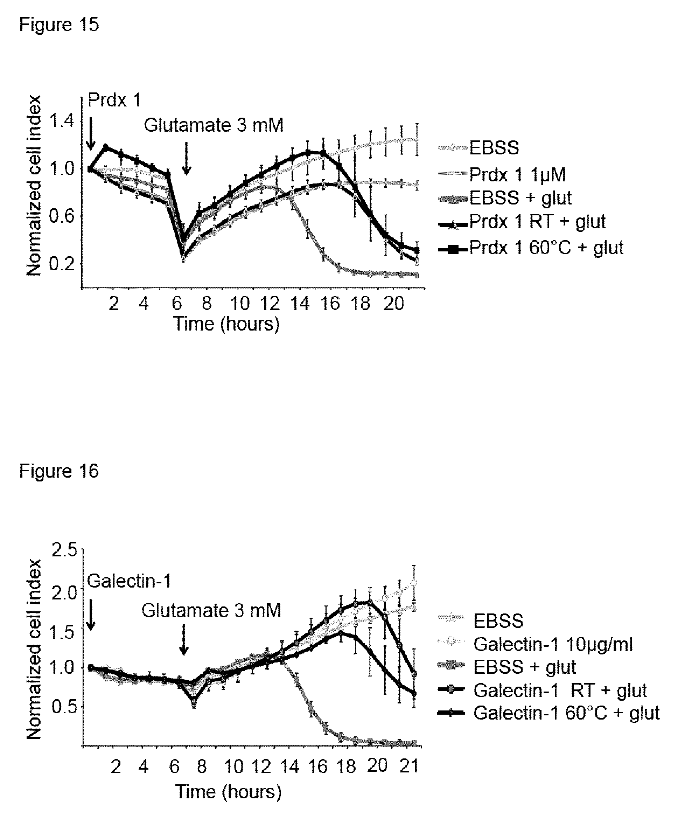

The neuroprotective effect of peroxiredoxin-1 (SEQ. ID No. 1) or galectin-1 (SEQ. ID No. 2) is tested by addition of 5 µm peroxiredoxin-1 (Prdx1) or 10 µg/ml galectin-1, respectively. As shown in Figures 15 and 16, respectively, both components provide a neuroprotective effect in growth factor deprived and glutamate-induced cells.

### Description of the drawings

**Figure 1.** Figure 1 shows a significant protective effect induced by NSPC-CM against growth factor withdrawal (-GF) and glutamate toxicity (40µM) in rat primary cortical neurons (n = 5).
**Figure 2.** Figure 2 illustrates that CM produced from dying NSPCs protects HT-22 cells against glutamate toxicity (3 mM, 15 hours) and growth factor withdrawal (21 hours) (n=8, ANOVA, Scheffé test).
**Figure 3.** Figure 3 shows HT-22 cells treated with CM produced from dying HT-22 cells by starvation. HT-22 CM show significant protective effect with and without heating to 60°C against glutamate (3 mM, 15 hours) and starvation (21 hours) (n = 6, ANOVA, Scheffé test).
**Figure 4.** Figure 4 demonstrates that SNL cells lysed by ultrasonic sound protect HT-22 cells against glutamate toxicity (3 mM, 15 hours) (n = 6; ANOVA, Scheffé test).
**Figure 5.** Figure 5 shows xCELLigence real time measurement. SNL and NSPC (designated as NPC) cells are lysed for 120 sec. Lysed NSPCs show significant protective effect against glutamate toxicity (3 mM) in HT-22 cells whereas SNL lysate had just a very short protective effect.
**Figure 6.** Figure 6 shows MTT cell viability analysis. HT-22 cells are pre-treated with CM from dying primary MEF. This CM fails to prevent HT-22 cells from glutamate neurotoxicity (3 mM) in MTT assay also after heat treatment at 60°C for 10 minutes (n = 7, ANOVA, Scheffé test).
**Figure 7.** Figure 7 shows MTT cell viability analysis. HT-22 cells are pre-treated with CM from dying SNL feeder cells. This CM fails to prevent HT-22 cells from glutamate neurotoxicity (3 mM) in MTT assay also after heat treatment at 60°C for 10 minutes (n = 7, ANOVA, Scheffé test).
**Figure 8.** Figure 8 illustrates that CM produced from NSPCs prevented from cell death by the broad caspase inhibitor Q-VD-OPh (20 µM) shows no protective effect from glutamate-induced apoptosis (3 mM, 15 hours) in HT-22 cells. Normal CM is used as a positive control (n= 6, ANOVA, Scheffé test).

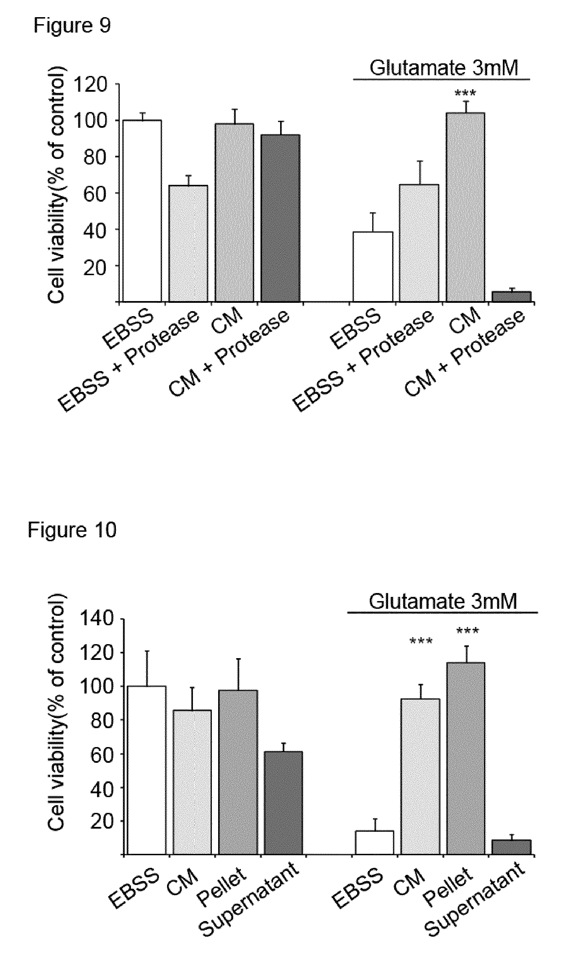

**Figure 9****.** CM pre-treated with protease fails to prevent glutamate-induced toxicity (3 mM, 15 hours) in HT-22 cells. CM without protease treatment is used as positive contol (n = 8, ANOVA, Scheffé test).
**Figure 10****.** Proteins in CM are precipitated by acetone. After separation by centrifugation precipitated proteins (pellet) show significant neuroprotection against glutamate damage (3 mM) in HT-22 cells. Supernatant fails to prevent cells from cell death in MTT test (n = 5, ANOVA, Scheffé test).
**Figure 11.** Figure 11 shows that proteins from CM precipitated by acetone show higher protective effect against glutamate damage after heat treatment for 10 minutes (n = 5, ANOVA, Scheffé test).
**Figure 12****.** CM pre-treated with RNAse fails to prevent glutamate-induced toxicity (3 mM) in HT-22 cells. CM without RNAse (n = 5) treatment is used as positive control.
**Figure 13.** Figure 13 demonstrates the heat activation of CM. Diluted NSPC-CM (1:4) was incubated at different temperatures for 30 min before pretreatment in HT22 cells. MTT assay demonstrates a protective effect against glutamate-induced neurotoxicity (3 mM) mediated by NSPC-CM heated up to 50 or up to 80°C but not 37°C, 90°C or 100°C (n = 6, ANOVA, Scheffé test).
**Figure 14****.** HT-22 cells are plated on an E-plate 96 and cellular impedance is continuously monitored by the xCELLigence system. CM heated up to 60°C for 10 minutes shows significant longer protection against glutamate (glut) toxicity (3 mM) compared to CM kept at room temperature (RT) (n = 3).
**Figure 15****.** Peroxiredoxin-1 is a protective component in CM. Recombinant human Peroxiredoxin-1 protects HT-22 cells against glutamate (glut, 3 mM) induced cell death with and without heating to 60°C (n = 3).
**Figure 16.** Figure 16 shows that recombinant mouse Galectin-1 protects HT-22 cells against glutamate (glut, 3 mM) induced cell death with and without heating to 60°C (n = 3).

The following sequences referred to herein are shown in the accompanying sequence listing:
**SEQ ID No. 1:** Peroxiredoxin-1
**SEQ ID No. 2:** Galectin-1

## Claims

1. A composition useful for treatment of acute injury or degenerative diseases comprising proteins from pluripotent neural stem and progenitor cells dying by cell death.

2. A composition according to claim 1, wherein the composition is derived from cell lysate of pluripotent neural stem and progenitor cells dying by cell death.

3. A composition according to claim 1, wherein the composition is derived from a conditioned medium of pluripotent neural stem and progenitor cells dying by cell death.

4. A composition according to one of claims 1 to 3, wherein the proteins from a conditioned medium are selected from the group of peroxiredoxins, galectins, heat shock proteins, transcription factors, and/or translation factors.

5. A method for producing the composition according to one of claims 1 to 4, comprising the steps:
i. cultivation of pluripotent neural stem and progenitor cells in appropriate cell culture medium,
ii. induction of cell death,
iii. harvest and lysis of cells or harvest of conditioned medium.

6. A method according to claim 5, wherein step iii. is followed by step iv. comprising filtration with a sterilization filter.

7. A method according to one of claims 5 or 6, wherein step iii. or iv. is followed by step v. comprising filtration with a filter having a size exclusion of at least 3 kDa.

8. A method according to one of claims 5 to 7, wherein step iii., iv. and/or v. is followed by step vi. comprising heating of the filtrate to between 20°C or up to 95°C for at least 10 minutes.

9. A method according to one of claims 5 to 8, wherein step iii., iv., v., and/or vi. is followed by step vii. comprising precipitation, fractionation, dialysis and/or lyophilisation.

10. A method according to one of claims 5 to 9, wherein the cell death is induced by growth factor deprivation, mechanical lysis, or heat shock.

11. Use of the composition according to one of claims 1 to 4 for manufacturing of a pharmaceutical composition for the treatment of acute injury or degenerative diseases of humans or animals.

12. Use of the composition according to claim 11 for manufacturing of a pharmaceutical composition, wherein the acute injury is caused by mechanical or ischemic injury to nerve cells or by irradiation.

13. Use of the composition according to claim 11 for manufacturing of a pharmaceutical composition, wherein the degenerative disease is a neurodegenerative disease of the peripheral nerve system or the central nerve system.

14. Use of the composition according to claim 11 for manufacturing of a pharmaceutical composition, wherein the degenerative disease is a cardiovascular disease.


SEQUENCE LISTING

<110>  Philipps-Universität Marburg

<120>  Stem cell derived composition for the treatment of acute injury
       and degenerative diseases

<130>  TM 651

<140>  EP 2012
<141>  2012-05-02

<160>  2

<170>  PatentIn version 3.5

<210>  1
<211>  199
<212>  PRT
<213>  Homo sapiens

<400>  1

Met Ser Ser Gly Asn Ala Lys Ile Gly His Pro Ala Pro Asn Phe Lys
1               5                   10                  15

Ala Thr Ala Val Met Pro Asp Gly Gln Phe Lys Asp Ile Ser Leu Ser
            20                  25                  30

Asp Tyr Lys Gly Lys Tyr Val Val Phe Phe Phe Tyr Pro Leu Asp Phe
        35                  40                  45

Thr Phe Val Cys Pro Thr Glu Ile Ile Ala Phe Ser Asp Arg Ala Glu
    50                  55                  60

Glu Phe Lys Lys Leu Asn Cys Gln Val Ile Gly Ala Ser Val Asp Ser
65                  70                  75                  80

His Phe Cys His Leu Ala Trp Val Asn Thr Pro Lys Lys Gln Gly Gly
                85                  90                  95

Leu Gly Pro Met Asn Ile Pro Leu Val Ser Asp Pro Lys Arg Thr Ile
            100                 105                 110

Ala Gln Asp Tyr Gly Val Leu Lys Ala Asp Glu Gly Ile Ser Phe Arg
        115                 120                 125

Gly Leu Phe Ile Ile Asp Asp Lys Gly Ile Leu Arg Gln Ile Thr Val
    130                 135                 140

Asn Asp Leu Pro Val Gly Arg Ser Val Asp Glu Thr Leu Arg Leu Val
145                 150                 155                 160

Gln Ala Phe Gln Phe Thr Asp Lys His Gly Glu Val Cys Pro Ala Gly
                165                 170                 175

Trp Lys Pro Gly Ser Asp Thr Ile Lys Pro Asp Val Gln Lys Ser Lys
            180                 185                 190

Glu Tyr Phe Ser Lys Gln Lys
        195

<210>  2
<211>  135
<212>  PRT
<213>  Homo sapiens

<400>  2

Met Ala Cys Gly Leu Val Ala Ser Asn Leu Asn Leu Lys Pro Gly Glu
1               5                   10                  15

Cys Leu Arg Val Arg Gly Glu Val Ala Pro Asp Ala Lys Ser Phe Val
            20                  25                  30

Leu Asn Leu Gly Lys Asp Ser Asn Asn Leu Cys Leu His Phe Asn Pro
        35                  40                  45

Arg Phe Asn Ala His Gly Asp Ala Asn Thr Ile Val Cys Asn Ser Lys
    50                  55                  60

Asp Gly Gly Ala Trp Gly Thr Glu Gln Arg Glu Ala Val Phe Pro Phe
65                  70                  75                  80

Gln Pro Gly Ser Val Ala Glu Val Cys Ile Thr Phe Asp Gln Ala Asn
                85                  90                  95

Leu Thr Val Lys Leu Pro Asp Gly Tyr Glu Phe Lys Phe Pro Asn Arg
            100                 105                 110

Leu Asn Leu Glu Ala Ile Asn Tyr Met Ala Ala Asp Gly Asp Phe Lys
        115                 120                 125

Ile Lys Cys Val Ala Phe Asp
    130                 135